# FASCICULE DE BREVET EUROPEEN

(11) **EP 0 820 722 B1**
(45) Date de publication et mention de la délivrance du brevet: **28.05.2003**
(21) Numéro de dépôt: 97450015.9
(22) Date de dépôt: 01.07.1997
(51) Int. Cl.: A61B 17/04, A61F 2/08

(54) **Vis médicale notamment pour la chirurgie et ancillaire de pose**
Chirurgische Schraube und Einsetzwerkzeug
Medical screw and installation tool for use in surgery

(30) Priorité: 02.07.1996 FR 9608442
(43) Date de publication de la demande: 28.01.1998
(73) Titulaire: Société Etudes et Developpements S.E.D. S.à.r.l., 19130 Voutezac (FR); Multi-Poles Conseils Société à Reponsabilité Limitée, 87600 Rochechouart (FR)
(72) Inventeur: Jammet, Jean, 19130 Voutezac (FR); Lenfant, Jean-Pierre, 87600 Rochechouart (FR); Peyre, André, 33000 Bordeaux (FR)
(74) Mandataire: Thébault, Jean-Louis

(56) Documents cités:
- WO-A-93/15666
- WO-A-96/28100
- FR-A- 2 704 171
- US-A- 5 002 550
- US-A- 5 176 682
- US-A- 5 250 055
- US-A- 5 324 308
- US-A- 5 443 482
- US-A- 5 503 634

## Description

La présente invention concerne une vis médicale utilisable notamment en chirurgie esthétique afin d'ancrer une suture chirurgicale ainsi que l'ancillaire de pose. L'invention présente également une variante de réalisation.

On connaît des vis ayant une fonction d'ancrage à usage médico-chirurgical notamment par le brevet US-A-5 443 482 qui décrit une vis auto taraudeuse dont le corps est surmonté d'une tête formant un oeilleton en sorte de permettre le passage d'un fil de fixation et même de plusieurs fils de fixation. L'inconvénient de cette vis est de nécessiter un ancillaire difficile à réaliser et difficile à mettre en oeuvre. De plus, l'oeilleton est d'un encombrement trop important compte tenu de l'application chirurgicale envisagée.

On connaît aussi le brevet WO-A-93 15666 qui décrit une vis percée d'un canal central débouchant, le fil de suture étant dans ce cas introduit à travers le canal et immobilisé par nouage à l'extrémité du canal. Cette vis présente l'avantage d'avoir un encombrement réduit mais le fil est pré-monté avec un noeud de blocage et définitivement immobilisé une fois la vis en place, il n'y a donc aucune possibilité de coulissement.

Un autre brevet US-A-5 411 523 décrit une vis qui est prévue pour être ancrée dans la corticale osseuse, cette vis étant surmontée d'une tige de manoeuvre venue de fabrication avec ladite vis. Cette tige est munie d'un point de rupture à l'interface vis/tige, en sorte de retirer la tige lorsque la vis est en place. La tige est par ailleurs creuse et sert de logement de stockage du fil de suture lié à la vis.

Hormis la fabrication dont on imagine aisément qu'elle est délicate, on peut noter deux inconvénients majeurs :
- le fil est fixe et ne peut coulisser, et
- la tige de manoeuvre pour le vissage, une fois désolidarisée de la vis ne peut être utilisée pour le démontage.

Le brevet US-A-5 176 682 décrit une cheville qui possède des ancrages latéraux sous forme de pattes déformables. Ces pattes sont venues de fabrication avec le corps de la cheville et résulte de découpes adaptées dans l'épaisseur de ce corps.

Une vis interne est prévue pour venir visser axialement dans le corps de ladite cheville et la pénétration de cette vis provoque la déformation des pattes qui viennent en saillie à l'extérieur du corps et s'ancrent dans la matière osseuse des parois d'un trou ménagé préalablement pour recevoir cette cheville. Une variante prévoit un clou à la place de la vis, le fonctionnement étant identique. On peut noter que le retrait de la cheville doit être relativement délicat voire impossible.

Par EP-A-0 814 708 - Article 54(3) CBE- on connaît une vis d'ancrage comportant en partie supérieure une tête de section carrée destinée à être reçue dans une empreinte carrée ménagée à l'extrémité distale d'un ancillaire. La mise en place d'une telle vis nécessite une préparation des abords textedu puits d'insertion, qui n'est pas facile et la vis une fois en place ne peut plus être extraite par dévissage.

Aussi, la présente invention se propose de présenter une vis à usage chirurgical, notamment pour servir d'ancrage à la fixation de tendons et autres ligaments, qui est d'un encombrement réduit, qui est simple, qui est fabriqué indépendamment de l'ancillaire, qui autorise un coulissement du fil de suture, qui nécessite un ancillaire particulier qui peut aisément être fabriqué pour le rendre à usage unique, et qui d'une mise en oeuvre très simple et toute naturelle pour le praticien avec des sécurités quant au guidage et au positionnement, l'ensemble autorisant aussi le retrait.

L'invention concerne aussi cet ancillaire particulier.

A cet effet, les vis médicales selon l'invention sont définies dans les revendications 1 à 3.

L'ancillaire est défini dans les revendications 4 à 8.

L'invention est décrite, ci-après, en regard des dessins annexés qui représentent des modes de réalisation particuliers, les différentes figures représentant:
- figure 1, une vue en coupe d'une vis selon l'invention et de l'ancillaire,
- figure 2, une vue en perpective de la vis selon l'invention,
- figure 3, une vue en coupe d'une vis en place dans la corticale osseuse avec le fil de suture apparent,
- figure 4, une vue en coupe suivant la ligne 4-4 de la figure 3,
- figure 5, une variante de réalisation de la vis,
- figure 6 une vue en coupe suivant la ligne 6-6 de la figure 5,
- figures 7A, 7B deux vues de mise en place du fil de suture dans le cas de la variante, avec un cheminement particulier du fil,
- figure 8, une vue de l'extrémité de l'ancillaire simplifié,
- figure 9A, une vue en coupe longitudinale d'une variante de mise en place du fil de suture, et
- figure 9B, une vue en coupe longitudinale perpendiculaire à celle de la figure 9A.

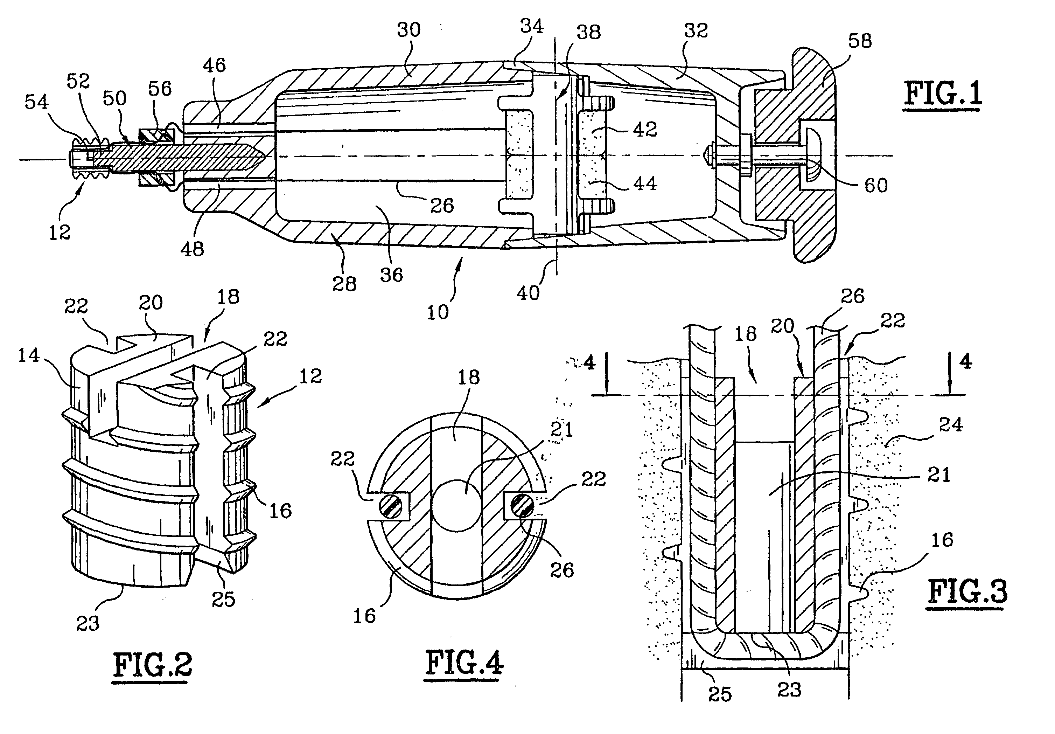

Sur la figure 1 on a représenté un ancillaire 10 et une vis 12 selon l'invention, en place sur le matériel ancillaire.

Sur la figure 2, on appréhende mieux les caractéristiques techniques de la vis 12 selon l'invention. En effet, cette vis comprend un corps de vis 14, plein, sensiblement cylindrique, sur la périphérie duquel est ménagé un filet 16.

Une rainure 18, débouchante, transversale et suivant un diamètre, est usinée à partir de la face supérieure 20 de la vis, avec dans la partie centrale de la rainure un trou 21, uniquement visible sur les figures 3 et 4.

Aux deux extrémités du diamètre perpendiculaire à la rainure 18, on trouve deux rainures longitudinales 22 usinées le long de deux génératrices. Ces deux rainures sont parfaitement polies en sorte de ne présenter aucune surface anguleuse.

En se reportant aux figures 3 et 4, on voit la vis implantée dans la matière osseuse 24, le filet 16 venant pénétrer dans la matière osseuse de façon connue.

Le fil chirurgical 26 passe dans l'une des rainures longitudinales 22, passe sous la face inférieure 23 de la vis pour ressortir par l'autre rainure longitudinale 22. La face inférieure 23 est munie d'une rainure 25, par exemple en vé, en sorte de faciliter le guidage du fil et de permettre le coulissement dudit fil même si la vis vient à fond de trou lors de la mise en place.

On constate que le fil peut coulisser librement entre la paroi osseuse et le fond de chacune des rainures 22.

La mise en place est réalisée à l'aide de l'ancillaire 10 représenté en coupe sur la figure 1, ancillaire qui comprend un corps creux 28 réalisé en deux parties 30 inférieure et 32 supérieure et prévues pour être solidarisées l'une à l'autre sensiblement au milieu du corps creux, en 34. Le corps creux comprend, une fois monté, un volume libre intérieur 36, dans lequel est logé un dévidoir 38 monté à rotation transversalement par rapport à l'axe longitudinal du corps creux, autour de l'axe 40. Ce dévidoir comprend deux enroulements 42 et 44 de fil de suture 26. A l'extrémité inférieure de la partie inférieure du corps creux, il est prévu deux trous 46,48 à travers lesquels passent les deux brins de fil de suture 26.

La partie inférieure 30 se prolonge par une lame tournevis 50 dont l'extrémité 52 est prévue pour coopérer avec la rainure 18. Cette extrémité 52 porte notamment un pion cylindrique de centrage 54 adapté pour pénétrer dans le trou 21 ménagé dans la partie centrale de la rainure 18.

Une bague 56, avec une fente en sifflet en partie médiane, pour servir de guidage et d'orientation, est montée coaxialement à la lame de tournevis 50, les brins du fil de suture 26 passant nécessairement entre l'extérieur de la lame tournevis et l'intérieur de ladite bague de façon à en assurer le guidage. On remarque aussi que la bague 56 permet de tendre le fil chirurgical, de le plaquer contre la lame tournevis pour qu'il se loge au fond des rainures longitudinales 22, le fil restant protégé lors de la mise en place de la vis.

L'extrémité supérieure de la partie supérieure 32 est surmontée d'un tourillon 58 monté libre en rotation grâce à un axe 60.

L'ancillaire, de type jetable, est donc réalisé de préférence en matière plastique pour des raisons évidentes de coût, la vis quant à elle étant réalisée en alliage léger, biocompatible, par exemple en titane ou alliage de titane.

Ainsi, l'ensemble vis et ancillaire est livré pré-monté.

Il suffit au praticien de percer avec un foret ayant sensiblement le diamètre du corps de vis, à fond de filet puis de présenter la vis maintenue à l'extrémité de l'ancillaire, de plaquer la vis à l'entrée du trou en appuyant sur le tourillon pour exercer un effort de pénétration avec, simultanément, une rotation du corps creux 28 de l'ancillaire. Cette rotation est transmise à la vis par la lame de tournevis.

Le praticien exerce une rotation jusqu'à ce que la vis ait parfaitement pénétré dans le trou puis il retire l'ancillaire et c'est à cet instant seulement que le fil enroulé autour du dévidoir se déroule. L'ancillaire peut alors être mis de côté jusqu'à la fin de l'intervention.

Le chirurgien procède à la fixation de l'élément biologique ligament, tendon ou muscle avec possibilité de faire coulisser le fil et d'ajuster parfaitement le positionnement. La vis quant à elle est parfaitement intégrée dans la matière osseuse sans élément en saillie.

En cas d'erreur de manipulation le chirurgien est à même de retirer la vis et le fil de suture qui y est attaché. Il suffit de replacer l'ancillaire, cette opération étant simplifiée par la bonne accessibilité de la rainure 18 sur la face supérieure 20 de la vis et grâce au pion de centrage 54 qui vient pénétrer dans le trou 21.

On remarque aussi que le filet 16 se trouve interrompu au droit de chacune des rainures longitudinales 22 formant autant de tarauds et facilitant ainsi la pénétration et la mise en place de la vis. Cette bonne pénétration des filets dans la matière osseuse assure un excellent ancrage.

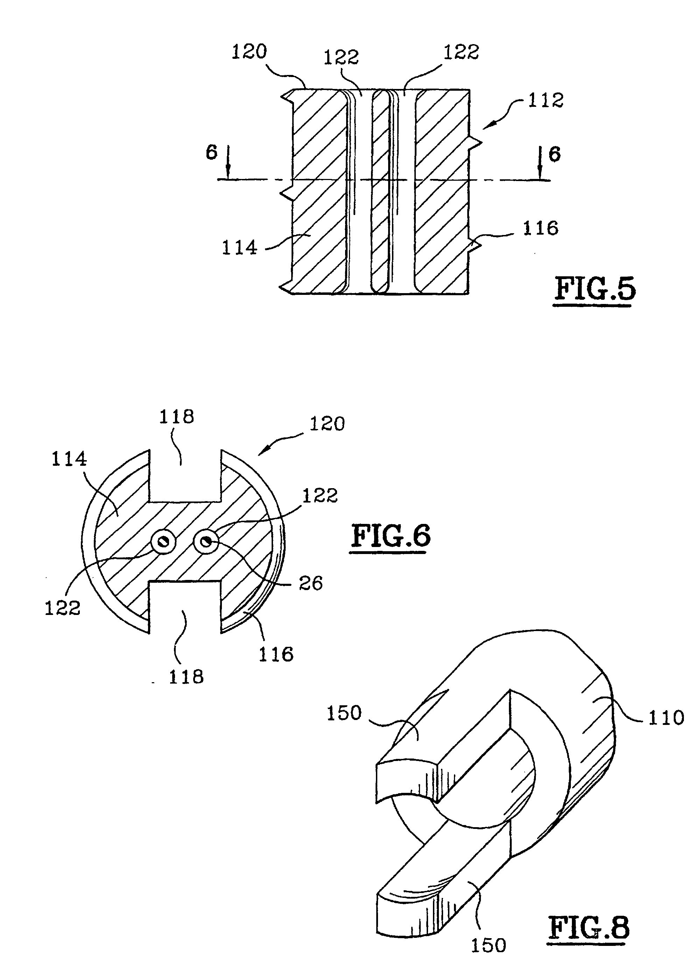

Une variante de l'invention est représentée sur les figures 5,6,7A et 7B, 8. Pour des raisons de simplification et de parallèle entre les deux variantes, les références portant sur des éléments identiques ou ayant la même fonction portent les mêmes références augmentées de 100.

La vis 120 de cette variante comprend deux trous 122 prévus pour le passage du fil 26. Ces deux trous sont disposés le long d'un premier diamètre et sur un second diamètre, perpendiculaire, on a ménagé deux rainures longitudinales 118 prévues pour recevoir les ergots 150 de vissage d'un ancillaire 110.

Le filet 116 est sensiblement identique au filet 16 précédent.

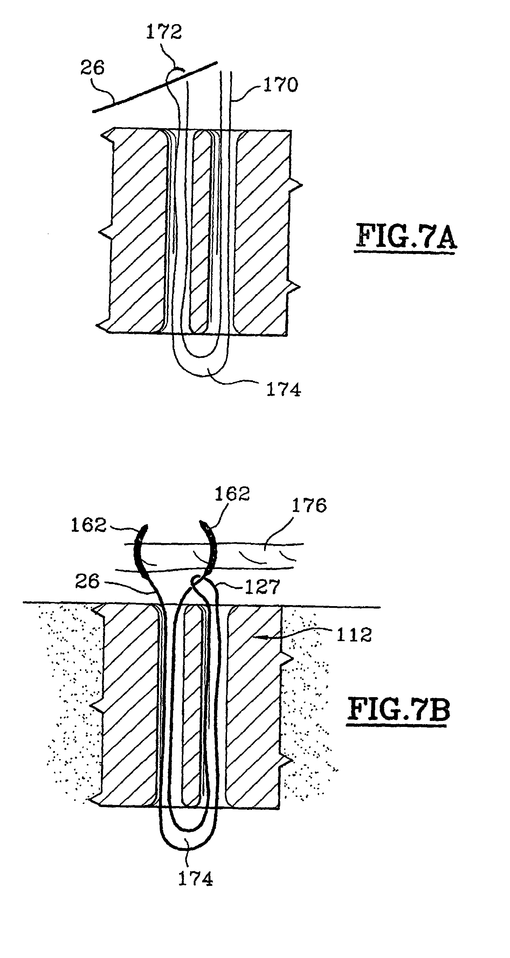

Dans cette variante, l'ancillaire 110 ne contient pas de fil et la vis contient une élingue 170, pré-montée, qui n'est autre qu'un fil métallique par exemple, de faible section et mis en forme pour pénétrer par un trou 122 et ressortir par l'autre trou 122, comme indiqué sur la figure 7A. L'élingue est double en sorte de former une boucle 172.

Les figures 7A et 7B montrent l'introduction et la mise en oeuvre d'un fil de suture 26 muni d'une aiguille 162 à chacune des extrémités, avec la vis selon la variante de réalisation.

Après vissage de la vis dans un trou réalisé de façon connue à l'aide d'un foret, il suffit d'introduire le fil de suture 26 dans la boucle 172 et de tirer sur l'élingue par ses deux extrémités libres simultanément. Le fil de suture suit alors l'élingue et se trouve introduit à son tour dans les deux trous 122 en passant par dessous la vis 112 et forme une boucle 174.

Le praticien est à même de fixer un élément référencé 176, sur cet ancrage au moyen du fil 26. Il lui suffit de piquer avec l'une des aiguilles 162 l'élément à fixer en le traversant, puis de passer le second brin avec son aiguille dans la boucle 127 du fil 26 avant de fixer ce second brin à l'élément 176, pour finir l'opération de fixation. Ce cheminement particulier du fil peut bien entendu s'appliquer au mode de réalisation principal.

Le praticien peut aussi faire passer le fil en simple et assurer la liaison de façon connue, le mode à boucle n'étant qu'une manière astucieuse de lier les fils.

On remarque de nouveau que le fil peut coulisser pour en ajuster le positionnement avant ou pendant la mise en place de l'élément 176. L'encombrement est réduit comme dans le cas précédent et l'élément peut être plaqué sans blessure sur l'os, au droit de la vis puisque aucun élément ne vient en saillie.

L'outil de mise en place est encore plus simple à réaliser avec la possibilité de recourir à une élingue et avec l'avantage de pouvoir choisir jusqu'au dernier moment le type de fil, mais cette variante de vis peut bien entendu être équipée pré-montée avec un fil de suture donné.

On remarque aussi que la mise à sa place de la vis est aisée grâce à un bon guidage par l'ancillaire.

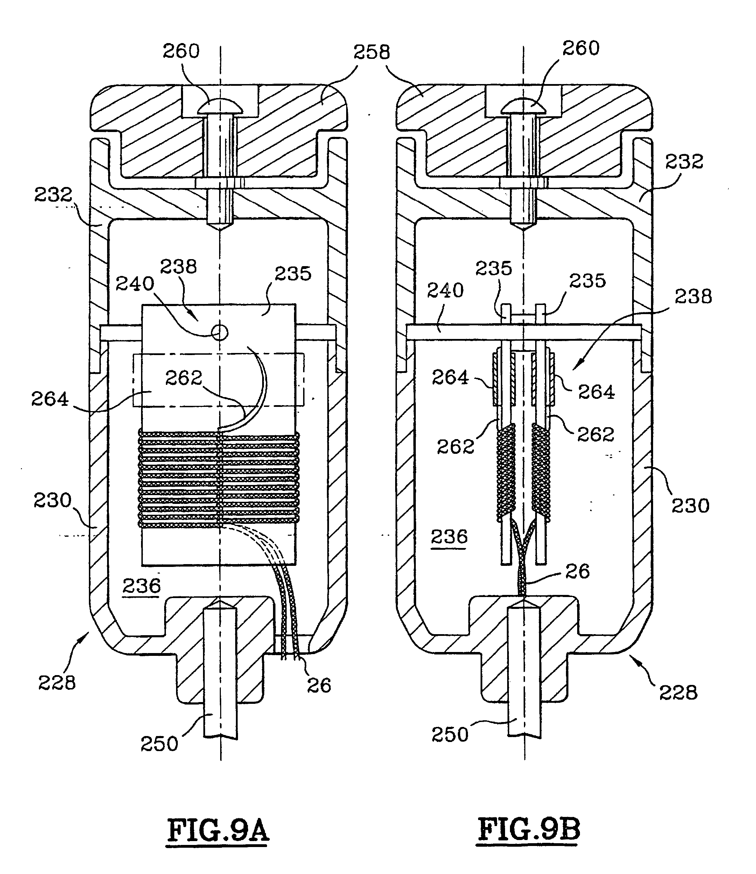

Sur les figures 9A et 9B, on a représenté une variante de réalisation de l'ancillaire, les éléments identiques ou ayant les mêmes fonctions sont référencés avec les mêmes nombres augmentés de 200.

Seul le dévidoir mobile en rotation est remplacé par deux plaquettes 235 sur lesquelles sont enroulés les longueurs libres du fil 26. Chaque plaquette est munie d'une fente dans laquelle le fil 26 de suture est freiné.

Un axe 240 de pivotement permet une bonne orientation des plaquettes 235, notamment lors du dévidage du fil.

On remarque aussi que dans cette variante, il est possible de disposer des aiguilles 262 à chacune des extrémités du fil sans que cela perturbe le dévidage du fil. Ces aiguilles sont maintenues par des bracelets élastiques 264 sur les plaques 235.

## Revendications

1. Vis médicale pour ancrage notamment dans la matière osseuse en chirurgie à l'aide d'un fil de suture (26) comprenant un corps (14,114) sensiblement cylindrique muni d'au moins un filet (16, 116) s'étendant depuis la face inférieure(23) jusqu'à la face supérieur (20) deux rainures (22) ménagées le long de deux génératrices opposées du corps de vis sur toute la longueur de ce dernier, destinées au passagelibre du fil de suture en boucle sous la vis, et une rainure transversale (18) ménagée sur ladite face supérieur (20) destinée à la réception d'un moyen de mise en rotation du corps de vis aussi bien pour la mise en place de la vis que son extraction.

2. Vis médicale suivant la revendication 1, **caractérisée en ce que** ladite face inférieure (23) du corps de vis est munie d'une rainure en vé (25) reliant les extrémités desdites rainures (22) ménagées le long de deux génératrices opposées, en sorte de permettre un libre coulissement du fil de suture même si la vis est en butée contre le fond du trou récepteur.

3. Vis médicale pour ancrage notammentdans la matière osseuse`en chirurgie à l'aide d'un fil de suture (174) comprenant un corps (14, 114) sensiblement cylindrique muni d'au moins un filet (16,116) s'étendant depuis la face inférieure (23) j'usqu'à la face supérieure (20) deux rainures (118) ménagées le long de deux génératrices opposées du corps de vis sur toute la longueur de ce dernier, destinées au passage d'ergots de vissage (150) d'un moyen de mise en rotation du corps de vis aussi bien pour lamise en place de la vis que son extraction, et deux trous longitudinaux (122) débouchants, disposés le long d'un diamètre perpendiculaire à celui reliant lesdites rainures ménagéesle long de deux génératrices, destinées au passage libre du fil de suture (174) en boucle dans la vis.

4. Ancillaire pour la mise en rotation de la vis médicale selon la revendication 1 ou 2, comprenant une lame (50) de tournevis prévue pour coopérer avec la rainure transversale (18) ainsi que des moyens de distribution et de stockage du fil (28,38,56).

5. Ancillaire suivant la revendication 4, **caractérisé en ce que** ladite lame (50) de tournevis est prolongée par un pion cylindrique de centrage (54) apte à pénétrer dans un trou (21) ménagé à cet effet dans la partie centrale de la rainure (18) de la vis.

6. Ancillaire suivant la revendication 4, **caractérisé en ce qu'**il comprend un corps creux (28) avec une ouverture (48) de passage du fil (26) de suture, dans lequel est logé un dévidoir (38) rotatif sur lequel sont enroulées les deux extrémités libres du fil de suture, avec une bague (56) disposée sur la lame (50) de tournevis en sorte de guider et de plaquer les brins du fil au fond des rainures (22) longitudinales.

7. Ancillaire suivant la revendication 4, **caractérisé en ce qu'**il comprend un corps creux (228) dans lequel sont logées deux plaques (235) sur lesquelles sont enroulées les deux extrémités libres du fil (26) de suture dont les extrémités comprennent éventuellement une aiguille (262).

8. Ancillaire suivant la revendication 6, **caractérisé en ce que** les plaques sont fendues pour assurer un freinage du fil et comprennent un bracelet élastique (264) pour le maintien des aiguilles.

## Patentansprüche

1. Medizinische Schraube für die chirurgische Verankerung insbesondere in Knochenmaterial mit Hilfe eines Nähfadens (26), mit einem Körper (14, 114), der im wesentlichen zylindrisch ist und wenigstens ein Gewinde (16, 116) aufweist, das sich von der unteren Oberfläche (23) bis zur oberen Oberfläche (20) erstreckt, zwei Nuten (22), die längs zweier gegenüberliegender Mantellinien des Schraubenkörpers auf seiner gesamter Länge ausgespart sind und dem freien Durchgang des Nähfadens in einer Schleife unter der Schraube dienen, und mit einer transversalen Nut (18), die auf der oberen Oberfläche (20) ausgespart ist und zur Aufnahme eines Mittels zum Drehen des Schraubenkörpers sowohl beim Einsetzen als auch beim Entfernen der Schraube vorgesehen ist.

2. Medizinische Schraube nach Anspruch 1, **dadurch gekennzeichnet, daß** die untere Oberfläche (23) des Schraubenkörpers mit einer prismaförmigen Nut (25) versehen ist, die die Enden der längs der gegenüberliegenden Mantellinien ausgesparten Nuten (22) derart verbindet, daß ein ungehindertes Gleiten des Nähfadens (26) ermöglicht wird, wenn die Schraube am Boden des aufnehmenden Lochs anliegt.

3. Medizinische Schraube für die chirurgische Verankerung, insbesondere in Knochenmaterial mit Hilfe eines Nähfadens (174), mit einem im wesentlichen zylindrischen Körper (14, 114), der mit mindestens einem Gewinde (16, 116) versehen ist, das sich von der unteren Oberfläche (23) bis zum oberen Oberfläche (20) erstreckt, zwei Nuten (118), die längs zweier gegenüberliegender Mantellinien des Schraubenkörpers auf seiner gesamten Länge ausgespart sind und dem Durchgang von Schraubvorsprüngen (150) eines Mittels zum Drehen des Schraubenkörpers sowohl beim Einsetzen der Schraube als auch bei ihrem Entfernen dienen, und mit zwei longitudinalen Durchtrittsöffnungen (122), die entlang eines Durchmessers angeordnet sind, der senkrecht zu demjenigen ist, der die beiden Nuten verbindet, die längs der beiden Mantellinien ausgespart sind, und dem ungehinderten Durchgang des Nähfadens (174) in einer Schleife in der Schraube dienen.

4. Hilfsgerät zum Drehen der medizinischen Schraube nach Anspruch 1 oder 2, mit einem Schraubendreherblatt (50), das dazu vorgesehen ist, mit der transversalen Nut (18) zusammenzuwirken sowie mit Mitteln für die Verteilung und Lagerung des Fadens (28, 38, 56).

5. Hilfsgerät nach Anspruch 4, **dadurch gekennzeichnet, daß** das Schraubendreherblatt (50) durch eine zylindrische Zentriereinrichtung (54) verlängert ist, geeignet zum Eingreifen in eine Aussparung (21), die hierfür im mittleren Abschnitt der Nut (18) der Schraube vorgesehen ist.

6. Hilfsgerät nach Anspruch 4, **dadurch gekennzeichnet, daß** es einen Hohlkörper (28) mit einer Öffnung (48) für den Durchgang des Nähfadens (26) umfaßt, in der eine drehbare Haspel (38) untergebracht ist, auf die die beiden freien Enden des Nähfadens aufgerollt sind, wobei auf dem Schraubendreherblatt (50) ein Ring (56) derart angeordnet ist, daß die Fasern des Nähfadens auf den Boden der longitudinalen Nuten (22) geführt und gegen diesen gedrückt werden.

7. Hilfsgerät nach Anspruch 4, **dadurch gekennzeichnet, daß** es einen Hohlkörper (228) umfaßt, in dem zwei Platten (235) untergebracht sind, auf die die beiden freien Enden des Nähfadens (26) aufgerollt sind, dessen Enden gegebenenfalls eine Nadel (262) aufweisen.

8. Hilfsgerät nach Anspruch 6, **dadurch gekennzeichnet, daß** die Platten geschlitzt sind, um eine Sicherung des Fadens zu gewährleisten, und ein elastisches Band (264) zum Halten der Nadeln umfassen.

## Claims

1. Medical screw for anchoring in particular in bony matter in surgery by means of a suture thread (26), comprising a substantially cylindrical body (14, 114) provided with at least one thread (16, 116) extending from the bottom face (23) as far as the top face (20), two grooves (22) provided along two opposite generatrices of the screw body over the entire length of the latter, intended for the free passage of the suture thread in a loop under the screw, and a transverse groove (18) provided on said top face (20) intended for receiving a means of rotating the screw body both for fitting the screw and for extracting it.

2. Medical screw according to claim 1, **characterised in that** said bottom face (23) of the screw body is provided with a V-shaped groove (25) connecting the ends of said grooves (22) formed along two opposite generatrices, so as to enable the suture thread (26) to slide freely even if the screw is in abutment against the bottom of the receiving hole.

3. Medical screw for anchoring in particular in bony matter in surgery by means of a suture thread (174), comprising a substantially cylindrical body (14, 114) provided with at least one thread (16, 116) extending from the bottom face (23) as far as the top face (20), two grooves (118) provided along two opposite generatrices of the screw body over the entire length of the latter, intended for the passage of lugs (150) for screwing means for rotating the screw body both for fitting the screw and extracting it, and two opening-out longitudinal holes (122), disposed along a diameter perpendicular to that connecting said grooves formed along two generatrices, intended for the free passage of the suture thread (174) in a loop in the screw.

4. Tool for rotating the medical screw according to claim 1 or 2, comprising a screwdriver blade (50) designed to cooperate with the transverse groove (18) as well as means for dispensing and storing the thread (28, 38, 56).

5. Tool according to claim 4, **characterised in that** said screwdriver blade (50) is extended by a cylindrical centring pin (54) able to enter a hole (21) provided for this purpose in the central part of the groove (18) in the screw.

6. Tool according to claim 4, **characterised in that** it comprises a hollow body (28) with an opening (48) for passage of the suture thread (26), in which there is housed a rotary pay-out device (38) on which the two free ends of the suture thread are wound, with a ring (56) disposed on the screwdriver blade (50) so as to guide and press the strands of the thread at the bottom of the longitudinal grooves (22).

7. Tool according to claim 4, **characterised in that** it comprises a hollow body (228) in which there are housed two plates (235) on which there are wound the two free ends of the suture thread (26), whose ends possibly comprise a needle (262).

8. Tool according to claim 6, **characterised in that** the plates are slotted to provide a retaining of the thread and comprise an elastic bracelet (264) for holding the needles.
